# EUROPEAN PATENT SPECIFICATION

(11) **EP 4 088 731 B1**
(45) Date of publication and mention of the grant of the patent: **06.11.2024**
(21) Application number: 20896503.8
(22) Date of filing: 04.12.2020
(51) Int. Cl.: A61K 38/10, A61K 38/08, A61K 38/17, A61P 19/02, A61P 29/00, A61P 17/06, A61P 1/00, A61P 11/06, A61P 37/00, A61K 35/583, A23L 33/18

(54) **COMPOSITION CONTAINING PEPTIDE OR PEPTIDE COMPOUND AS ACTIVE INGREDIENT, AND MEDICAL USE THEREFOR**
ZUSAMMENSETZUNG MIT PEPTID ODER PEPTIDVERBINDUNG ALS WIRKSTOFF UND DEREN MEDIZINISCHE VERWENDUNG
COMPOSITION CONTENANT UN PEPTIDE OU UN COMPOSÉ PEPTIDIQUE UTILISÉ COMME PRINCIPE ACTIF, ET SON UTILISATION MÉDICALE

(30) Priority: 05.12.2019 KR 20190160690; 05.12.2019 KR 20190160691; 26.11.2020 KR 20200160761
(43) Date of publication of application: 16.11.2022
(73) Proprietor: Ajou University Industry-Academic Cooperation Foundation, Suwon-si, Gyeonggi-do 16499 (KR)
(72) Inventor: SOHN, Seonghyang, Suwon-si Gyeonggi-do 16699 (KR)
(74) Representative: Roos, Peter
(86) International application number: PCT/KR2020/017629
(87) International publication number: WO 2021/112615

(56) References cited:
- WO-A1-2015/087334
- WO-A1-2018/209127
- WO-A1-99/29726
- KR-B1- 101 802 515
- DATABASE PROTEIN 7 April 2021 (2021-04-07), ANONYMOUS: "RecName: Full=Weak toxin S4C11", XP055818075, retrieved from NCBI Database accession no. P01400
- WANG SHU-ZHI, QIN ZHENG-HONG: "Anti-Inflammatory and Immune Regulatory Actions of Naja naja atra Venom", TOXINS, vol. 10, no. 3, 28 February 2018 (2018-02-28), XP055818117, DOI: 10.3390/toxins10030100

## Description

### TECHNICAL FIELD

The present disclosure relates to a composition which contains peptides or a mixture thereof as an active ingredient for preventing or treating a disease selected from the group consisting of psoriasis, inflammatory bowel disease, Crohn's disease, allergic asthma, atopic dermatitis, and systemic inflammatory response syndrome induced by an autoimmune response.

### BACKGROUND ART

Human immune system plays a role in protecting the body from foreign antigens invading the human body, without attacking its own tissues owing to self-tolerance. However, a case called autoimmunity occurs when self-tolerance of the immune system breaks down to make immune cells to produce antibodies recognizing proteins that are normally expressed by own genes as a target to attack, or to destroy normal tissues by causing T-cell response. When specific symptoms appear, it is called an autoimmune disease.

Inflammatory response is a recovery mechanism that attempts to regenerate damaged areas caused by chemical substances, bacterial infection, and physical action that bring about organic changes in living body or tissues. When inflammation occurs by harmful stimuli, infection, or external injury, vasoactive substances such as inflammatory components are locally released to cause inflammation. Such persisting inflammation may rather promote mucosal damage, thereby developing inflammatory diseases such as rheumatoid arthritis, arteriosclerosis, gastritis, and asthma due to rubefaction, fever, swelling, pain, and dysfunction.

Various mediators are involved in the inflammatory response, including cytokines, prostaglandins E2 (PGE2), and free radicals. In particular, nuclear factor-κB which is a transcription factor involved in the inflammatory response is activated in macrophages by stimuli such as cytokines, tumor necrosis factor-α (TNF-α), and lipopolysaccharide (LPS). As a result, inducible nitric oxide synthase (iNOS) and cyclooxygenase-2 (COX-2) are expressed, and excess nitric oxide (NO) and prostaglandin E2 produced thereby causes inflammation.

Psoriasis is a non-contagious, chronic, inflammatory skin disease characterized by hyperproliferative keratinocytes and an increase in scaly patches. Although the cause of disease is still unknown, it is a skin disease caused by immunological abnormalities due to chronic activation of inflammatory cell infiltration in the skin and dysregulation of epidermal keratinocytes. According to the results in research, psoriasis is known as one of the autoimmune diseases developed with involvement of T lymphocytes, and it is also reported that the main cause of psoriasis development is excessive activation of T lymphocytes.

Inflammatory bowel disease is a disease that causes chronic inflammation in the bowel with unknown causes and may be classified into ulcerative colitis and Crohn's disease. Both ulcerative colitis and Crohn's disease are chronic intractable diseases with recurring symptoms though once improved temporarily. Although the cause or pathophysiology of inflammatory bowel disease is yet to be known clearly, it is estimated that a combination of genetic factors, environmental factors such as intestinal bacteria or food, and immunological factors may be involved in the development mechanism. Despite rapidly increasing incidence rate of ulcerative colitis and Crohn's disease, a fundamental treatment has not yet been established due to an unclear cause. Therefore, drugs that delay and alleviate the progression of symptoms are being applied rather than the fundamental treatment.

### DISCLOSURE

### TECHNICAL GOALS

An object of the present disclosure is to provide a pharmaceutical composition for preventing or treating a disease selected from the group consisting of psoriasis, inflammatory bowel disease, Crohn's disease, allergic asthma, atopic dermatitis, and systemic inflammatory response syndrome induced by an autoimmune response, by providing a composition comprising peptides or a mixture thereof as an active ingredient.

### TECHNICAL SOLUTIONS

Example embodiments of the present disclosure provide a pharmaceutical composition for preventing or treating a disease selected from the group consisting of psoriasis, inflammatory bowel disease, Crohn's disease, allergic asthma, atopic dermatitis, and systemic inflammatory response syndrome, comprising a peptide having an amino acid sequence represented by SEQ ID NO: 1, a peptide having an amino acid sequence represented by SEQ ID NO: 2, a peptide having an amino acid represented by SEQ ID NO: 3, a peptide having an amino acid represented by SEQ ID NO: 4, a peptide having an amino acid represented by SEQ ID NO: 5, or a mixture thereof as an active ingredient.

In addition, example embodiments of the present disclosure provide a health food for preventing or alleviating a disease selected from the group consisting of psoriasis, inflammatory bowel disease, Crohn's disease, allergic asthma, atopic dermatitis, and systemic inflammatory response syndrome, comprising a peptide having an amino acid sequence represented by SEQ ID NO: 1, a peptide having an amino acid sequence represented by SEQ ID NO: 2, a peptide having an amino acid represented by SEQ ID NO: 3, a peptide having an amino acid represented by SEQ ID NO: 4, a peptide having an amino acid represented by SEQ ID NO: 5, or a mixture thereof as an active ingredient.

### ADVANTAGEOUS EFFECTS

According to example embodiments of the present disclosure, P1 to P5 peptides or a mixture thereof were intraperitoneally administered or applied in the form of external skin preparations to a mouse animal model with psoriasis and inflammatory bowel disease which are inflammatory diseases induced by an autoimmune response. As a result, it was found that skin ulcers and skin inflammation were alleviated in the mouse model with psoriasis, and symptoms of inflammation were reduced in the model with enteritis. Thus, it is possible to provide a composition comprising the P1 to P5 peptides or a mixture thereof as an active ingredient as a therapeutic agent or a health food for diseases induced by autoimmune response.

### BRIEF DESCRIPTIONS OF THE DRAWINGS

FIG. 1 shows a result of FACS analysis identifying the frequency of CD40+ cells and CD83+ cells which are markers for dendritic cell activation among peripheral blood cells after administration of a peptide mixture to a mouse animal model with psoriasis.
FIG. 2 shows a result of FACS analysis identifying the frequency of RORyt-expressing cells among peripheral blood cells after administration of a peptide mixture to a mouse animal model with psoriasis.
FIG. 3 shows a result of FACS analysis identifying the frequency of regulatory T cells among peripheral blood cells after administration of a peptide mixture to a mouse animal model with psoriasis.
FIG. 4 shows a result of FACS analysis identifying the frequency of Ly6G-expressing neutrophils among abdominal macrophages after administration of a peptide mixture to a mouse animal model with psoriasis.
FIG. 5 shows a result of FACS analysis identifying the frequency of CD8+NK1.1+ cells among lymphocytes isolated from lymph nodes after administration of a peptide mixture to a mouse animal model with psoriasis.
FIG. 6 shows results of checking symptoms of inflammation after administration of a control, a control cream, and a peptide P124 in the form of external skin preparations for 5 days to the skin of a mouse animal model with psoriasis.
FIG. 7 shows results of observing the change in the size of spleen after oral administration of a peptide mixture to a mouse animal model with enteritis induced with dextran sulfate sodium (DSS).
FIG. 8 shows results of FACS analysis identifying the frequency of CD40+ cells and CD83+ cells which are markers for dendritic cell activation among peripheral blood cells and the frequency of CD83+ cells and CD86+ cells among intraperitoneal macrophages, after oral administration of a peptide mixture to a mouse animal model with enteritis.
FIG. 9 shows results of FACS analysis identifying the frequency of CD11b+ cells as a marker for macrophages among intraperitoneal macrophages, the frequency of NK1.1+ cells as a marker for natural killer cells among splenocytes, the frequency of cells expressing Ly6G+ as a marker for neutrophils, and the frequency of cells expressing ROR gammaT as a gene involved in Th17+ cell differentiation, after oral administration of a peptide mixture to a mouse animal model with enteritis.
FIG. 10 shows a result of identifying the frequency of fluorescence-labeled cells among intraperitoneal macrophages 48 and 72 hours after oral administration of peptides 1, 2, and 4 each conjugated with different fluorescence to normal mice.

### BEST MODE

Hereinafter, an example embodiment of the present disclosure will be described in more detail.

An example embodiment of the present disclosure may provide a pharmaceutical composition for preventing or treating a disease selected from the group consisting of psoriasis, inflammatory bowel disease, Crohn's disease, allergic asthma, atopic dermatitis, and systemic inflammatory response syndrome, comprising a peptide having an amino acid sequence represented by SEQ ID NO: 1, a peptide having an amino acid sequence represented by SEQ ID NO: 2, a peptide having an amino acid represented by SEQ ID NO: 3, a peptide having an amino acid represented by SEQ ID NO: 4, a peptide having an amino acid represented by SEQ ID NO: 5, or a mixture thereof as an active ingredient.

The peptide may prevent or alleviate inflammation induced by an autoimmune response.

The pharmaceutical composition may be a formulation selected from the group consisting of oral preparations, external preparations, suppositories, and injections, but is not limited thereto.

In an example embodiment of the present disclosure, the pharmaceutical composition may further include one or more additives selected from the group consisting of carriers, excipients, disintegrants, sweeteners, coating agents, swelling agents, glydents, flavoring agents, antioxidants, buffers, bacteriostatic agents, diluents, dispersants, surfactants, binders, and lubricants appropriate to be commonly used in the preparation for pharmaceutical compositions.

Specifically, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil may be used as the carriers, excipients and diluents. Solid preparations for oral administration may include tablets, pills, powder, granules and capsules, wherein such solid preparation may be prepared by mixing at least one excipient in the composition, for example, starch, calcium carbonate, sucrose or lactose, and gelatin. In addition, lubricants such as magnesium stearate and talc may be used in addition to simple excipients. A liquid formulation for oral dosage may include suspensions, solutions, emulsions, and syrups, and various excipients such as wetting agents, sweeteners, fragrances, and preservatives may be included in addition to water and liquid paraffin, which are commonly used simple diluents. The formulation for parenteral administration may include sterile aqueous solutions, non-aqueous solvents, suspensions, emulsions, freeze-dried formulations, and suppositories. Propylene glycol, polyethylene glycol, vegetable oils such as olive oil, and injectable esters such as ethyl oleate may be used as the non-aqueous solvents and suspensions. Witepsol, macrogol, Tween 61, cacao butter, laurin fat, and glycerogelatin may be used as a base of the suppositories.

According to an example embodiment of the present disclosure, the pharmaceutical composition may be administered to a subject in a conventional manner via intravenous, intraarterial, intraperitoneal, intramuscular, intrasternal, transdermal, intranasal, inhalational, topical, rectal, oral, intraocular or intradermal routes.

The preferred dosage of the peptide may vary depending on condition and weight of the subject, the type and severity of the disease, the drug form, and the route and duration of administration, and may be appropriately selected by those skilled in the art. According to an example embodiment of the present disclosure, although not limited thereto, the daily dose may be 0.01 µg/kg to 200 mg/kg, specifically 0.1 to 200 mg/kg, more specifically 0.1 to 100 mg/kg. Administration may be performed once a day or several times in divided doses, but not limiting the scope of the present disclosure thereby.

In an example embodiment of the present disclosure, the 'subject' may be a mammal including a human, but is not limited to the examples.

An example embodiment of the present disclosure provides a health food for preventing or alleviating a disease selected from the group consisting of psoriasis, inflammatory bowel disease, Crohn's disease, allergic asthma, atopic dermatitis, and systemic inflammatory response syndrome, comprising a peptide having the amino acid sequence represented by SEQ ID NO: 1, a peptide having the amino acid sequence represented by SEQ ID NO: 2, a peptide having the amino acid represented by SEQ ID NO: 3, a peptide having the amino acid represented by SEQ ID NO: 4, a peptide having the amino acid represented by SEQ ID NO: 5, or a mixture thereof as an active ingredient.

The health food may be used together with other food or food additives in addition to the peptide or a mixture thereof, and may be appropriately used according to a conventional method. The compound amount of the active ingredient may be appropriately determined depending on the intended use thereof, for example, prevention, health or therapeutic treatment.

The effective dose of the compound contained in the health food may be applied according to the effective dose of the therapeutic agent, but in the case of long-term intake for health and hygiene or health control, it may be less than or equal to the above range. However, it is clear that the active ingredient may be used in an amount beyond the above range since there is no problem in terms of safety.

The type of health food is not particularly limited, and examples include meat, sausage, bread, chocolate, candy, snacks, confectionery, pizza, ramen, other noodles, gum, dairy products including ice cream, various soups, beverages, tea, drinks, alcoholic beverages, and vitamin complexes.

### MODES FOR CARRYING OUT INVENTION

Hereinafter, examples will be described in detail to help the understanding of the present disclosure. However, the following examples are merely illustrative of the content of the present disclosure, and the scope of the present disclosure is not limited to the following examples. The examples of the present disclosure are provided to more completely explain the present disclosure to those skilled in the art.

### <Example 1> Preparation of peptides

With efforts to develop a low-molecular substance for the treatment of inflammation from snake venom, particularly a substance known as a protein, peptides with medicinal effects have been discovered. Peptides having the following 11 to 23 amino acid sequences were prepared.
P1: LICPEKYCNKVHT (SEQ ID NO: 1)
P2: YCNKVHTCRNG (SEQ ID NO: 2)
P3: PREIVECCSTDKCNH (SEQ ID NO: 3)
P4: HTCRNGENICF (SEQ ID NO: 4)
P5: ENICFKRFYEGNLLGKRYPRGCA (SEQ ID NO: 5)

### <Example 2> Identification of effect of peptides in mouse model with psoriasis

20 µg/day/mouse of imiquimod (IMQ) was administrated to C56BL/6J mice as an external skin preparation once a day for 6 days to induce symptoms of psoriasis.

A peptide mixture (P124) and the control cream were administrated daily for 5 days as an external skin preparation to check characteristics of immune cells, wherein the peptide mixture was obtained by mixing peptides P1, P2, and P4 isolated in Example 1 with physiological saline for injection so as to be 1 µg respectively.

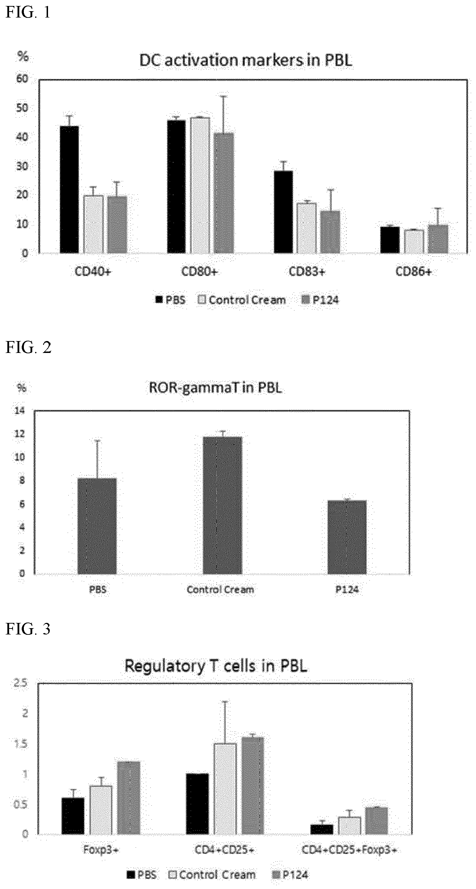

As a result of analyzing peripheral blood cells by flow cytometry (FACS) after administration of the peptide, it was found that the frequency of CD40 positive (+) cells and CD83+ cells which are markers for dendritic cell activation decreased as shown in FIG. 1. In addition, a decrease in the frequency of cells expressing RORyt was detected as shown in FIG. 2. RORyt is a marker for Th17 cells, and an increase in Th17 cells is associated with aggravation of psoriasis.

In addition, as shown in FIG. 3, it was found that the frequency of regulatory T cells having a function of suppressing the inflammatory response increased in the peptide-administered group.

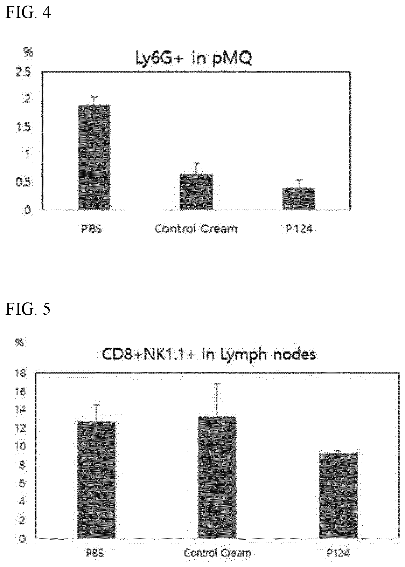

As a result of analyzing the intraperitoneal macrophages by flow cytometry (FACS) after administration of the peptides, it was found that the frequency of neutrophils expressing Ly6G decreased as shown in FIG. 4. Excessive expression of neutrophils is known to cause excessive inflammatory response due to excessive secretion of cytokines.

As a result of analyzing lymphocytes isolated from lymph nodes by flow cytometry (FACS) after administration of peptides, it was found that the frequency of CD8+NK1.1+ cells decreased as shown in FIG. 5. CD8+NK1.1+ cells secrete IFNγ and are involved in innate immune response to control microbial pathogens. However, excessive expression thereof is known to be associated with induction of inflammation.

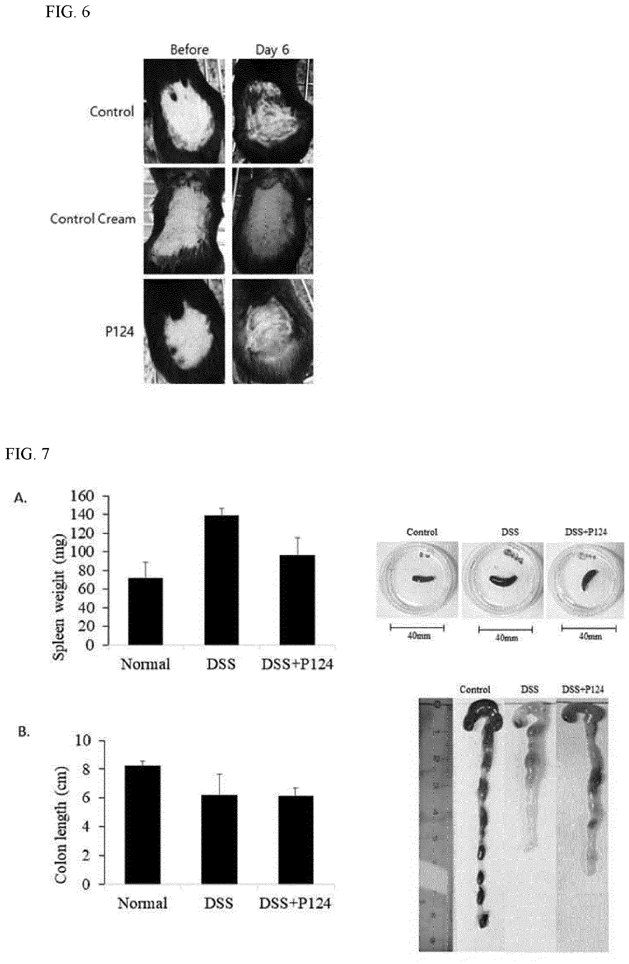

However, as a result of comparing symptomatic changes after daily administration of the control, the control cream, and the peptide mixture (P124) as external skin preparations for 5 days to a mouse animal model induced with psoriasis symptoms, inflammatory symptoms appeared on the skin after 6 days in the control and control cream group as shown in FIG. 6, but it was found that psoriasis symptoms barely remained in the peptide-treated group.

### <Example 3> Identification of effect of peptides in animal model with inflammatory bowel disease

The peptide mixture (P124) obtained by mixing the peptides P1, P2 and P4 isolated in Example 1 with physiological saline for injection to be 1 µg respectively was orally administered to a mouse animal model with enteritis induced by dextran sulfate sodium (DSS) once a day for 4 consecutive days.

As a result of spleen extraction in mice orally administered with the peptide mixture (P124), it was found that the size of spleen was reduced as shown in FIG. 7.

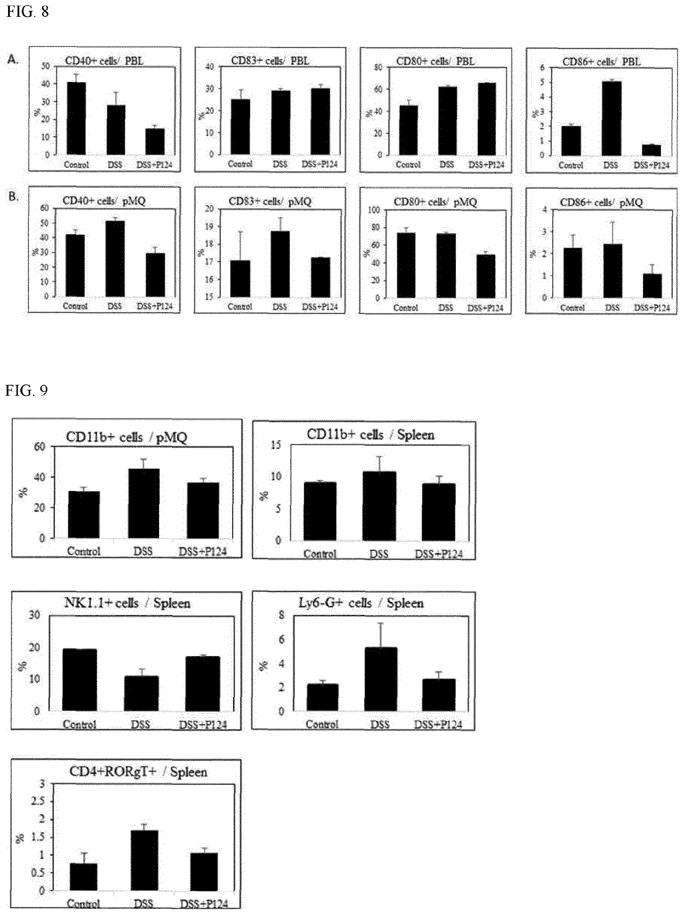

In addition, as a result of observing the immune cells in the mouse model with enteritis with the peptide mixture administered, the frequency of CD40+ dendritic cells and CD86+ dendritic cells decreased among peripheral blood cells as shown in FIG. 8, and the frequency of CD83+ cells and CD86+ cells also decreased among intraperitoneal macrophages due to administration of the peptides.

It is known that overexpression of dendritic cell activation markers is associated with prolonged chronic inflammation, and it was found that peptide administration decreased the dendritic cell activation markers from above results, thereby alleviating symptoms of inflammatory bowel diseases through administration of the peptide.

In addition, as shown in FIG. 9, it was found that the frequency of CD11b+ cells as a macrophage marker decreased among intraperitoneal macrophages after administration of the peptide to the mouse model with enteritis, and a decrease in the frequency of CD11b+ cells was observed in splenocytes as well. In splenocytes, the frequency of NK1.1+ cells as a marker for natural killer cells increased, and the frequency of cells expressing Ly6G+ cells as a marker for neutrophils decreased. The frequency of cells expressing ROR gammaT, a gene involved in Th17+ cell differentiation, decreased.

From the results above, it was found that the peptide of the present disclosure may alleviate symptoms of colitis by decreasing the frequency of inflammation inducing cells.

Furthermore, peptides 1, 2, and 4 were orally administered to normal mice after conjugation with each different fluorescence, and the frequency of fluorescence-labeled cells was measured among intraperitoneal macrophages after 48 and 72 hours.

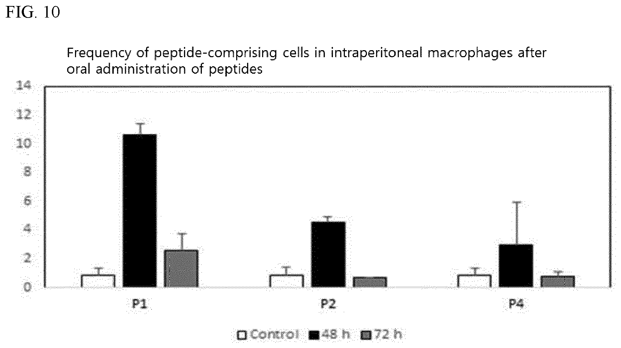

As a result, as shown in FIG. 10, P2 and P4 peptides were maintained up to 48 hours, and P1 was maintained up to 72 hours.

From the above results, it was found that the orally administered peptide reached the intraperitoneal cavity to be involved in the immune response, thereby inducing the alleviation of symptoms of colitis.

Although specific parts of the present disclosure have been described in detail above, it is clear for those skilled in the art that these specific descriptions are merely preferred example embodiments and the scope of the present disclosure is not limited thereto. Accordingly, the substantial scope of the present disclosure may be defined by the appended claims and equivalents thereof.

## Claims

1. A pharmaceutical composition for use in preventing or treating a disease selected from the group consisting of psoriasis, inflammatory bowel disease, Crohn's disease, allergic asthma, atopic dermatitis, and systemic inflammatory response syndrome, comprising a peptide comprising an amino acid sequence represented by SEQ ID NO: 1, a peptide comprising an amino acid sequence represented by SEQ ID NO: 2, a peptide comprising an amino acid represented by SEQ ID NO: 3, a peptide comprising an amino acid represented by SEQ ID NO: 4, a peptide comprising an amino acid represented by SEQ ID NO: 5, or a mixture thereof as an active ingredient.

2. The pharmaceutical composition for use of claim 1, wherein the peptide prevents or alleviates inflammation induced by an autoimmune response.

3. The pharmaceutical composition for use of claim 1, wherein the pharmaceutical composition is a formulation selected from the group consisting of oral preparations, external preparations, suppositories, and injections.

4. A health food for use in preventing or alleviating a disease selected from the group consisting of psoriasis, inflammatory bowel disease, Crohn's disease, allergic asthma, atopic dermatitis, and systemic inflammatory response syndrome, comprising a peptide comprising an amino acid sequence represented by SEQ ID NO: 1, a peptide comprising an amino acid sequence represented by SEQ ID NO: 2, a peptide comprising an amino acid represented by SEQ ID NO: 3, a peptide comprising an amino acid represented by SEQ ID NO: 4, a peptide comprising an amino acid represented by SEQ ID NO: 5, or a mixture thereof as an active ingredient.

## Patentansprüche

1. Pharmazeutische Zusammensetzung zur Verwendung bei der Vorbeugung oder Behandlung einer Krankheit, die ausgewählt ist aus der Gruppe bestehend aus Psoriasis, entzündlicher Darmerkrankung, Morbus Crohn, allergischem Asthma, atopischer Dermatitis und systemischem Entzündungsreaktionssyndrom, umfassend ein Peptid umfassend eine Aminosäuresequenz, die durch SEQ ID NO: 1 dargestellt ist, ein Peptid umfassend eine Aminosäuresequenz, die durch SEQ ID NO: 2 dargestellt ist, ein Peptid umfassend eine Aminosäure, die durch SEQ ID NO: 3 dargestellt ist, ein Peptid umfassend eine Aminosäure, die durch SEQ ID NO: 4 dargestellt ist, ein Peptid umfassend eine Aminosäure, die durch SEQ ID NO: 5 dargestellt ist, oder eine Mischung davon, als einen Wirkstoff.

2. Pharmazeutische Zusammensetzung zur Verwendung nach Anspruch 1, wobei das Peptid eine durch eine Autoimmunreaktion induzierte Entzündung verhindert oder lindert.

3. Pharmazeutische Zusammensetzung zur Verwendung nach Anspruch 1, wobei die pharmazeutische Zusammensetzung eine Formulierung ist, die ausgewählt ist aus der Gruppe bestehend aus oralen Präparaten, Präparaten zur äußerlichen Anwendung, Suppositorien und Injektionen.

4. Gesundheitsnahrungsmittel zur Verwendung bei der Vorbeugung oder Linderung einer Krankheit, die ausgewählt ist aus der Gruppe bestehend aus Psoriasis, entzündlicher Darmerkrankung, Morbus Crohn, allergischem Asthma, atopischer Dermatitis und systemischem Entzündungsreaktionssyndrom, umfassend ein Peptid umfassend eine Aminosäuresequenz, die durch SEQ ID NO: 1 dargestellt ist, ein Peptid umfassend eine Aminosäuresequenz, die durch SEQ ID NO: 2 dargestellt ist, ein Peptid umfassend eine Aminosäure, die durch SEQ ID NO: 3 dargestellt ist, ein Peptid umfassend eine Aminosäure, die durch SEQ ID NO: 4 dargestellt ist, ein Peptid umfassend eine Aminosäure, die durch SEQ ID NO: 5 dargestellt ist, oder eine Mischung davon, als einen Wirkstoff.

## Revendications

1. Composition pharmaceutique pour utilisation dans la prévention ou le traitement d'une maladie choisie parmi le groupe constitué par le psoriasis, les maladies inflammatoires de l'intestin, la maladie de Crohn, l'asthme allergique, la dermatite atopique et le syndrome de réponse inflammatoire systémique, comprenant un peptide comprenant une séquence d'acides aminés représentée par SEQ ID NO : 1, un peptide comprenant une séquence d'acides aminés représentée par SEQ ID NO : 2, un peptide comprenant un acide aminé représenté par SEQ ID NO : 3, un peptide comprenant un acide aminé représenté par SEQ ID NO : 4, un peptide comprenant un acide aminé représenté par SEQ ID NO : 5, ou un mélange de ceux-ci, en tant qu'ingrédient actif.

2. Composition pharmaceutique pour utilisation selon la revendication 1, dans laquelle le peptide prévient ou atténue l'inflammation induite par une réponse auto-immune.

3. Composition pharmaceutique pour l'utilisation selon la revendication 1, dans laquelle la composition pharmaceutique est une formulation choisie parmi le groupe constitué par les préparations orales, les préparations externes, les suppositoires et les injections.

4. Aliment de santé pour utilisation dans la prévention ou l'atténuation d'une maladie choisie parmi le groupe constitué par le psoriasis, les maladies inflammatoires de l'intestin, la maladie de Crohn, l'asthme allergique, la dermatite atopique et le syndrome de réponse inflammatoire systémique, comprenant un peptide comprenant une séquence d'acides aminés représentée par SEQ ID NO : 1, un peptide comprenant une séquence d'acides aminés représentée par SEQ ID NO : 2, un peptide comprenant un acide aminé représenté par SEQ ID NO : 3, un peptide comprenant un acide aminé représenté par SEQ ID NO : 4, un peptide comprenant un acide aminé représenté par SEQ ID NO : 5, ou un mélange de ceux-ci, en tant qu'ingrédient actif.
